# EUROPEAN PATENT APPLICATION

(11) **EP 2 123 313 A1**
(43) Date of publication of application: **25.11.2009**
(21) Application number: 07713571.3
(22) Date of filing: 09.03.2007
(51) Int. Cl.: A61M 1/14, B29C 45/10, B29C 45/14, B29C 45/26, B29L 22/00

(54) **METHOD OF FORMING BODY FLUID PURIFICATION CASSETTE**

(71) Applicant: Asahi Kasei Kuraray Medical Co., Ltd., Chiyoda-ku Tokyo 101-8101 (JP)
(72) Inventor: TAKESAWA, Shingo, Tokyo 142-0064 (JP)
(74) Representative: von Kreisler Selting Werner
(86) International application number: PCT/JP2007/000189
(87) International publication number: WO 2008/111111

(57) **Abstract**

The present invention provides a forming method capable of inexpensively forming a bodily fluid purification cassette which is reduced in size as small as possible. A bodily fluid purification cassette (U1) is detachably attached to a bodily fluid purification apparatus body. The bodily fluid purification cassette (U1) includes a synthetic resin box-like body (F1, F2); a basic element (R1, 1, PT1) incorporated in the box-like body; a selective element (2) selected from a bodily fluid purification element such as a dialyzer (2), a plasma exchange module and an immunoadsorbent cylinder; and bodily fluid flow passages (R1 to R7) which connects the basic element and the selective element with each other. The box-like body (F1, F2) and the bodily fluid flow passages (R1 to R7) are formed by primary molding such that a cross section of a split-type structure has a predetermined shape, and the split-type structure is integrally formed into a cassette by secondary molding. When the bodily fluid flow passages of the split-type structure are integrally formed by secondary molding, the basic element (R1, 1, PT1) and the selective element (2) are liquid-tightly connected to the bodily fluid flow passages (R1 to R7).

## Description

### Technical Field

The present invention relates to a forming method of a bodily fluid purification cassette in which the bodily fluid purification cassette is selectively attached to a bodily fluid purification apparatus body comprising a bubble detector which detects bubbles in a flow passage, a pressure detector which detects a pressure of bodily fluid in the flow passage, a dialysate fluid supply device, a heater, a thermometer, a conductivity meter, a pump rotor of at least one roller type pump, and a control apparatus, the bodily fluid purification cassette incorporates: basic elements comprising at least a bodily fluid flow passage, an air trap and a fluid feed pump tube; a selective element selected from bodily fluid purification elements comprising a dialyzer, a blood filter, a precise filter, a mesh filter, a plasma exchange module and an immunoadsorbent cylinder; and a bodily fluid flow passage which connects the basic element and the selective element with each other, and if the bodily fluid purification cassette which incorporates these elements is attached to the bodily fluid purification apparatus body, the pump rotor of the bodily fluid purification apparatus body cooperates with the fluid feed pump tube of the basic element to constitute a roller type pump, so that desired bodily fluid purification such as blood dialysis, peritoneal dialysis, plasma exchange and immune adsorption can be carried out.

### Background Technique

A treating apparatus or a purification apparatus of bodily fluid such as blood is conventionally known as a peritoneal dialysis apparatus, blood dialysis apparatus, a blood dialysis filtering apparatus and the like, and patent document 1 proposes a blood dialysis apparatus for example. Patent document 2 discloses a blood purification apparatus.

Patent document 1: Japanese Patent Application Laid-open No.2003-305118
Patent document 2: Japanese Patent Application Laid-open No.H9-239024

The blood dialysis apparatus shown in patent document 1 includes a blood dialysis element, i.e., a dialyzer. The dialyzer is provided therein with a dialysis film such as to divide the dialyzer into two in its axial direction, one of them is a blood flow passage and the other one is a dialysate fluid flow passage. Therefore, if blood flows through the blood flow passage and dialysate fluid flows through the dialysate fluid flow passage such that they are opposed to each other, the blood is subjected to dialysis treatment as conventionally known. To subject the blood to the dialysis treatment by the dialyzer, a filter, an air trap, a blood pump, a dialysate fluid supplying and receiving element, an on-off valve, a flowmeter, a control apparatus and the like are also required. Although these elements are not clearly explained in patent document 1, they are accommodated in a casing together with the dialyzer, and a relatively large blood dialysis apparatus is constituted.

The blood purification apparatus proposed in patent document 2 has a blood purification device having a permeable membrane. Blood taken out from a patient is introduced into the blood purification device, and blood from which blood component is filtered is returned to the patient together with displacing solution. At that time, the blood component or filtered liquid is discharged out from a body together with dialysate fluid. To purify blood, a blood pump, a filtered-liquid pump, a replacement liquid pump, a dialysate fluid pump, a control apparatus and the like are also required, they are accommodated in one casing, and the blood purification apparatus is constituted.

### Disclosure of the Invention

### Problem to be Solved by the Invention

In the blood dialysis apparatus proposed in patent document 1, the above-described various elements required for dialysis treatment of blood are accommodated in a casing together with the dialyzer, and a plurality of elements are accommodated in one casing of the blood purification apparatus disclosed in patent document 2 together with the blood purification device and thus, various merits can be obtained. For example, if a plurality of kinds of bodily fluid treating apparatuses such as blood dialysis apparatus and blood purification apparatus are installed in medical facility, a patient can carry out blood permeating treatment and purifying treatment if necessary. Further, a plurality of patients having different diseases can receive treatments at the same time by the respective bodily fluid treating apparatuses. There is also a merit that since the apparatus is a special apparatus, a medical specialist can handle the apparatus easily.
However, there is also a problem or drawback. For example, in order to handle a plurality of patients having different diseases, a plurality of various bodily fluid treating apparatuses must be installed and thus, there is a drawback that the cost is increased. For example, although the number of patients who require dialysis of blood and the number of patients who required purification of blood is not always the same, two kinds of bodily fluid treating apparatuses must be installed. That is, the bodily fluid treating apparatus which has different frequency in use and which is not frequently used must be installed also and thus, the cost is increased. Further, there is a problem in terms of space where a plurality of bodily fluid treating apparatuses are installed. Due to the problem of cost, there is a problem that a small medical facility can not install a bodily fluid treating apparatus for a small number of patients, and the patients must go to a medical facility located far away.

The present invention provides a bodily fluid treating apparatus by which the conventional problems are solved. To solve the problems, a blood dialysis apparatus can be divided into a first portion mainly comprising a dialyzer in terms of effect, and a second portion comprising a bubble detector which detects bubbles in a flow passage through which bodily fluid flows, a pressure gauge which measures a pressure value, a dialysate fluid supply device, a heater, a thermometer, a control apparatus and the like. A blood purification apparatus can also be divided into a first portion mainly comprising a blood purification device, and a second portion comprising a blood pump, a filtered-liquid pump, a liquid-supply pump, a dialysate fluid pump, a control apparatus and the like. It can be found that the second portion of the blood dialysis apparatus and the second portion of the blood purification apparatus have elements which have different names but have the same functions or exhibit the same effects. Hence, the second portions are accommodated in an apparatus body, the first portions are constituted as a bodily fluid purification cassette or a unit, the bodily fluid purification cassette is attached to the apparatus body so that they can carry out different bodily fluid treatments. With this, the conventional problem concerning cost and the problem concerning the installing space can be solved.
Therefore, it is an object of the present invention to inexpensively provide a forming method of a bodily fluid purification cassette which is reduced in size as small as possible and which can be formed as the bodily fluid purification cassette. It is also an object of the invention to provide a forming method capable of automatically form in a sanitary manner. It is also an object of the invention to provide a forming method of a bodily fluid purification cassette suitable for disposable structure.

### Means for Solving the Problems

To achieve the above objects, according to the present invention, basic elements comprising a bodily fluid flow passage, an air trap, and a fluid feed pump tube; and selective elements selected from bodily fluid purification elements comprising a dialyzer, a blood filter, a precise filter, a mesh filter, a plasma exchange module, an immunoadsorbent cylinder; are incorporated in a synthetic resin hollow container. A bodily fluid flow passage which connects the basic element and the selective element and the hollow container are formed as split-type structures by primary molding, and the split-type structures are integrally formed by secondary molding. To achieve the above object, the invention described in claim 1 provides a forming method of a bodily fluid purification cassette in which the bodily fluid purification cassette is selectively attached to a bodily fluid purification apparatus body comprising a bubble detector which detects bubbles in a flow passage, a pressure detector which detects a pressure of bodily fluid in the flow passage, a dialysate fluid supply device, a heater, a thermometer, a conductivity meter, a pump rotor of at least one roller type pump, and a control apparatus, the bodily fluid purification cassette incorporates: basic elements comprising at least a bodily fluid flow passage, an air trap and a plurality of fluid feed pump tubes; a selective element selected from bodily fluid purification elements comprising a dialyzer, a blood filter, a precise filter, a mesh filter, a plasma exchange module and an immunoadsorbent cylinder; and a bodily fluid flow passage which connects the basic element and the selective element with each other, and if the bodily fluid purification cassette which incorporates these elements is attached to the bodily fluid purification apparatus body, the pump rotor of the bodily fluid purification apparatus body cooperates with the fluid feed pump tube of the basic element to constitute a roller type pump, so that desired bodily fluid purification such as blood dialysis, peritoneal dialysis, plasma exchange and immune adsorption can be carried out, wherein a synthetic resin hollow container in which the basic element, the selective element and the bodily fluid flow passage are incorporated, and the bodily fluid flow passage are formed such that a cross section of a split-type structure has a predetermined shape by primary molding, the split-type structure is integrally formed by secondary molding to form the bodily fluid purification cassette in which the basic element, the selective element and the bodily fluid flow passage are incorporated in the synthetic resin hollow container, and when the bodily fluid purification cassette is formed, concerning the bodily fluid flow passage, a pair of first and second semi-molded articles having substantially semi-circular cross section of the split-type structure and a partition member having substantially a plate-like shape are injection molded by primary molding such that the pair of first and second semi-molded articles are provided at their butting portions with coupling flanges, the butting portions of the pair of first and second semi-molded articles sandwich the partition member by secondary molding, molten resin is injected into a junction space constituted by the flange portions of the pair of first and second semi-molded articles, the pair of first and second semi-molded articles and the partition member are liquid-tightly integrally formed together, and the bodily fluid flow passage comprising two flow passages is formed.
The invention described in claim 2 provides a forming method of a bodily fluid purification cassette in which the bodily fluid purification cassette is selectively attached to a bodily fluid purification apparatus body comprising a bubble detector which detects bubbles in a flow passage, a pressure detector which detects a pressure of bodily fluid in the flow passage, a dialysate fluid supply device, a heater, a thermometer, a conductivity meter, a pump rotor of at least one roller type pump, and a control apparatus, the bodily fluid purification cassette incorporates: basic elements comprising at least a bodily fluid flow passage, an air trap and a plurality of fluid feed pump tubes; a selective element selected from bodily fluid purification elements comprising a dialyzer, a blood filter, a precise filter, a mesh filter, a plasma exchange module and an immunoadsorbent cylinder; and a bodily fluid flow passage which connects the basic element and the selective element with each other, and if the bodily fluid purification cassette which incorporates these elements is attached to the bodily fluid purification apparatus body, the pump rotor of the bodily fluid purification apparatus body cooperates with the fluid feed pump tube of the basic element to constitute a roller type pump, so that desired bodily fluid purification such as blood dialysis, peritoneal dialysis, plasma exchange and immune adsorption can be carried out, wherein a synthetic resin hollow container in which the basic element, the selective element and the bodily fluid flow passage are incorporated, and the bodily fluid flow passage are formed such that a cross section of a split-type structure has a predetermined shape by primary molding, the split-type structure is integrally formed by secondary molding to form the bodily fluid purification cassette in which the basic element, the selective element and the bodily fluid flow passage are incorporated in the synthetic resin hollow container, and when the bodily fluid purification cassette is formed, the bodily fluid flow passage is formed by primary molding such that the bodily fluid flow passage includes a pair of first and second semi-molded articles of split-type structure having substantially a semi-circular cross section, butting portions thereof have flange portions and ends of the flange portions have coupling step having increased diameter, connected portions of the basic element and the selective element are inserted into the coupling steps of the first and second semi-molded articles and the molds are clamped, molten resin is injected by secondary molding into a junction space constituted by the flange portions of the first and second semi-molded articles, and into a space between outer peripheries of the connected portions and inner peripheries of the coupling steps of the first and second semi-molded articles, and the basic element and the selective element are liquid-tightly connected to the bodily fluid flow passage.
According to the invention described in claim 3, in the forming method of claim 1 or 2, the primary molding and the secondary molding are carried out using the same mold, and the bodily fluid purification cassette is formed. According to the invention described in claim 4, in the forming method of claim 2, the basic element and the selective element are preheated in the same mold before the secondary molding.
According to the invention described in claim 5, in the forming method of any one of claims 1 to 4, when the first and second semi-molded articles are to be formed by the primary molding, they are formed so that a cross section shape of the junction space constituted by the flange portions by butting the butting portions against each other has substantially a triangular shape whose angle portions are rounded, and so that an inner peripheral surface side of the butting portion is recessed radially outward such that its cross section has substantially a triangular shape. According to the invention described in claim 6, in the forming method of any one of claims 1 to 5, the basic element, the selective element and the bodily fluid flow passage are formed such that they can be accommodated in a synthetic resin hollow container having a size equal to or less than 220mm in vertical length, 300mm in lateral length and 80mm in height.

### Advantageous Effects

According to the present invention, the bodily fluid purification cassette has a synthetic resin hollow container, the container includes basic elements comprising at least a bodily fluid flow passage, an air trap and a fluid feed pump tube; a selective element selected from bodily fluid purification elements comprising a dialyzer, a blood filter, a precise filter, a mesh filter, a plasma exchange module and an immunoadsorbent cylinder; and a bodily fluid flow passage which connects the basic element and the selective element with each other, the bodily fluid flow passage are formed such that a cross section of a split-type structure has a predetermined shape by primary molding, the split-type structure is integrally formed by secondary molding to form the bodily fluid purification cassette in which the basic element, the selective element and the bodily fluid flow passage are incorporated in the synthetic resin hollow container. When the bodily fluid purification cassette is formed, concerning the bodily fluid flow passage, a pair of first and second semi-molded articles having substantially semi-circular cross section of the split-type structure and a partition member having substantially a plate-like shape are injection molded by primary molding such that the pair of first and second semi-molded articles are provided at their butting portions with coupling flanges, the butting portions of the pair of first and second semi-molded articles sandwich the partition member by secondary molding, molten resin is injected into a junction space constituted by the flange portions of the pair of first and second semi-molded articles, the pair of first and second semi-molded articles and the partition member are liquid-tightly integrally formed together, and the bodily fluid flow passage comprising two flow passages is formed. That is, since the bodily fluid purification cassette is formed by the injection molding, it can be automatized easily, and it can be formed inexpensively. Further, since it is formed by the mold, it can be formed in a sanitary manner. As described above, according to the present invention, it is possible to obtain effect which is unique to the invention that the bodily fluid purification cassette can inexpensively and automatically be formed or produced in a sanitary manner. The bodily fluid flow passage obtained by this forming method can be utilized as reciprocating roads or two-way roads, and there is an effect that the bodily fluid purification cassette can further be reduced in size.
According to another invention, the bodily fluid flow passage of the bodily fluid purification cassette is formed by primary molding such that the bodily fluid flow passage includes a pair of first and second semi-molded articles of split-type structure having substantially a semi-circular cross section, butting portions thereof have flange portions and ends of the flange portions have coupling step having increased diameter, connected portions of the basic element and the selective element are inserted into the coupling steps of the first and second semi-molded articles and the molds are clamped, molten resin is injected by secondary molding into a junction space constituted by the flange portions of the first and second semi-molded articles, and into a space between outer peripheries of the connected portions of the basic element and the selective element and inner peripheries of the coupling steps of the first and second semi-molded articles, and the basic element and the selective element are liquid-tightly connected to the bodily fluid flow passage. Therefore, in addition to the above effect, there is a further effect that the basic element and the selective element are liquid-tightly integrally formed with the bodily fluid flow passage when the bodily fluid flow passage is formed. Further, according to the invention for preheating the basic element and the selective element in the same mold before the secondary molding, heat which must cool the mold for taking out the molded article is effectively utilized to preheat, and the basic element and the selective element can be connected to the bodily fluid flow passage while saving energy. Further, when the first and second semi-molded articles are to be formed by the primary molding, they are formed so that a cross section shape of the junction space constituted by the flange portions by butting the butting portions against each other has substantially a triangular shape whose angle portions are rounded, and so that an inner peripheral surface side of the butting portion is recessed radially outward such that its cross section has substantially a triangular shape. According to this invention, the junction space is expanded or deformed by the pressure of resin which is charged into the junction space at the time of secondary molding, and the inner peripheral surface sides of the butting portions are brought into contact with each other under pressure. With this, a bodily fluid flow passage having extremely small gap between the butting portions, or having no gap therebetween can be obtained.

### Brief Description of the Drawings

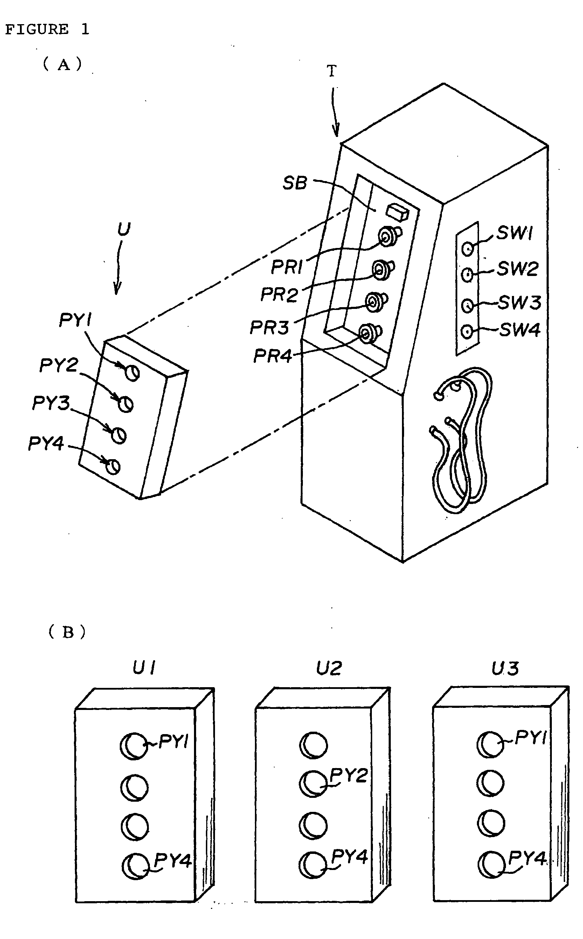

Figs. 1 show a bodily fluid purification apparatus, wherein (A) is a perspective view of a bodily fluid purification apparatus body on which a bodily fluid purification cassette formed or produced by carrying out the present invention, and (B) is a perspective view showing a plurality of different bodily fluid purification cassettes;

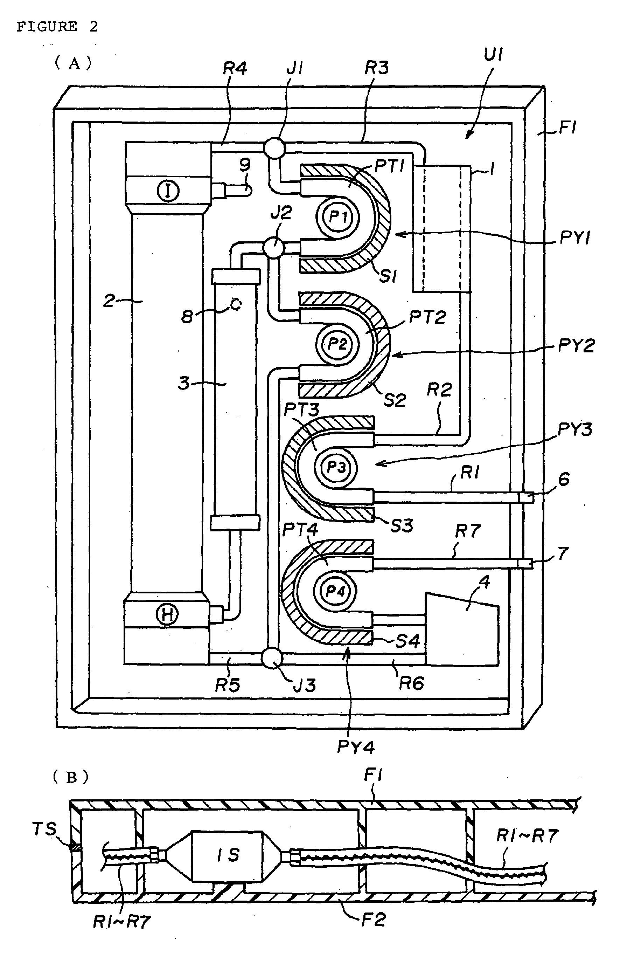

Figs. 2 are schematic diagrams showing a blood purification cassette formed by carrying out the invention, wherein (A) is a plan view thereof and (B) is a sectional view thereof;

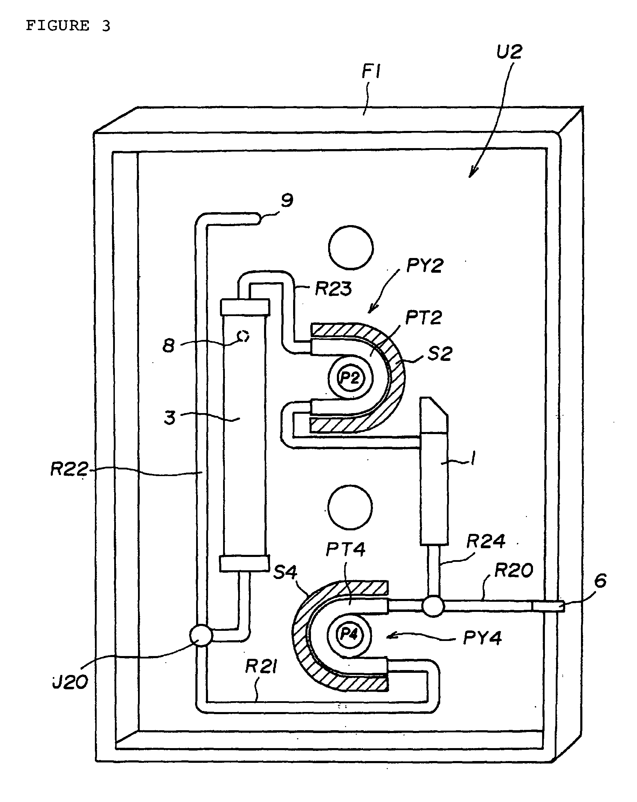

Fig. 3 is a schematic plan view of a peritoneal purification cassette formed by carrying out the invention;

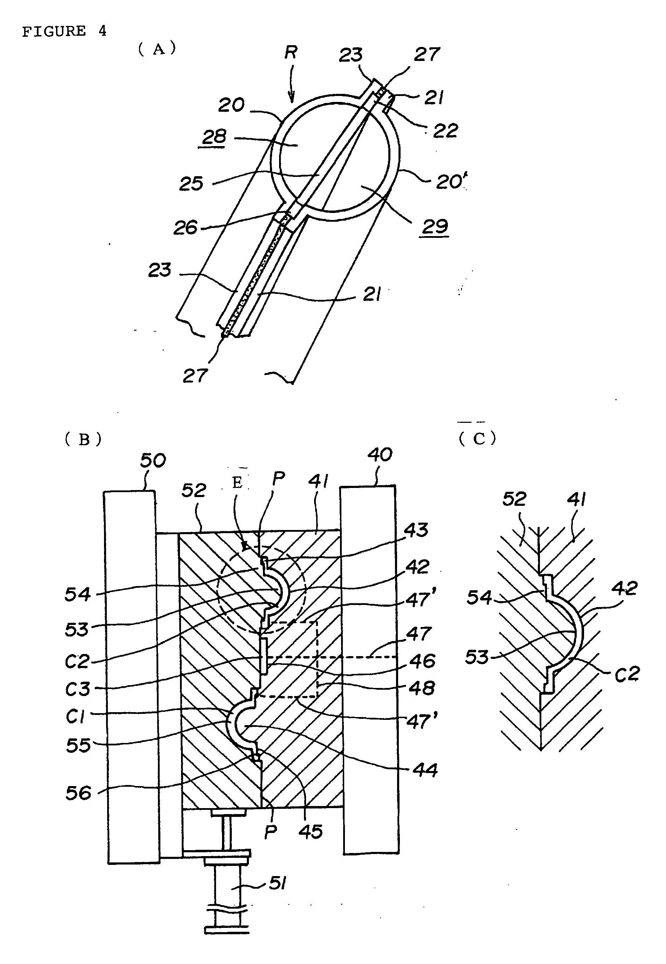

Figs. 4 show an embodiment of the invention, wherein (A) is a perspective view of a bodily fluid flow passage obtained by carrying out the invention, (B) is a sectional view of a mold used for carrying out the invention, and (C) is an enlarged sectional view of a portion shown with the arrow E in (B);

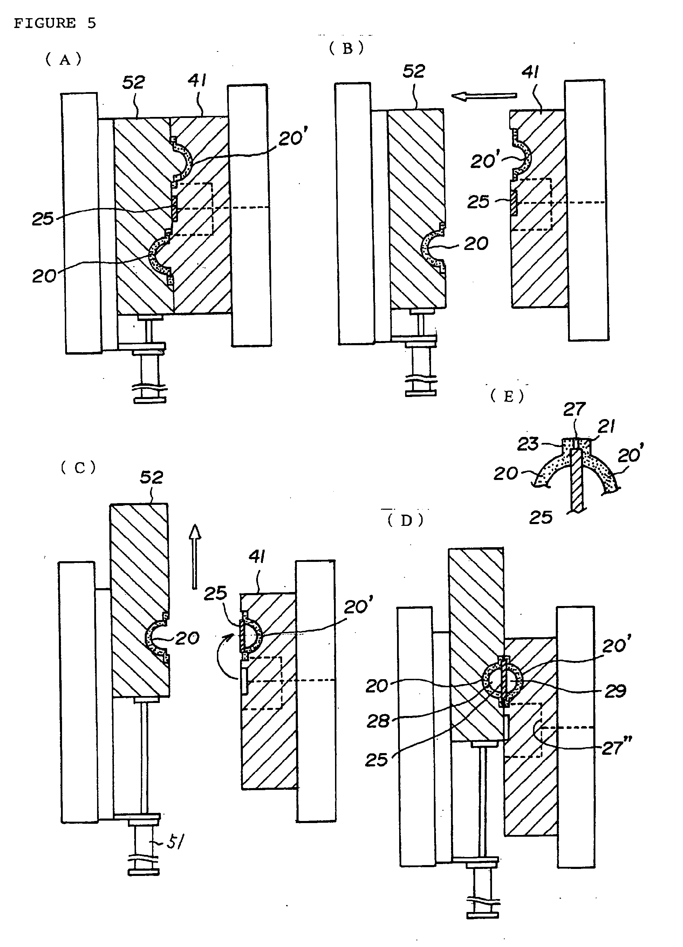

Figs. 5 are schematic diagrams of a forming example of the bodily fluid flow passage shown in Fig. 4(A), wherein (A) shows a state where primary molding is completed, (B) shows a state where a slide mold is opened, (C) shows a state where the slide mold is slid toward the secondary molding position, (D) is a sectional view showing a state where the secondary molding is completed, and (E) is an enlarged sectional view of butting portions of a pair of semi-molded article;

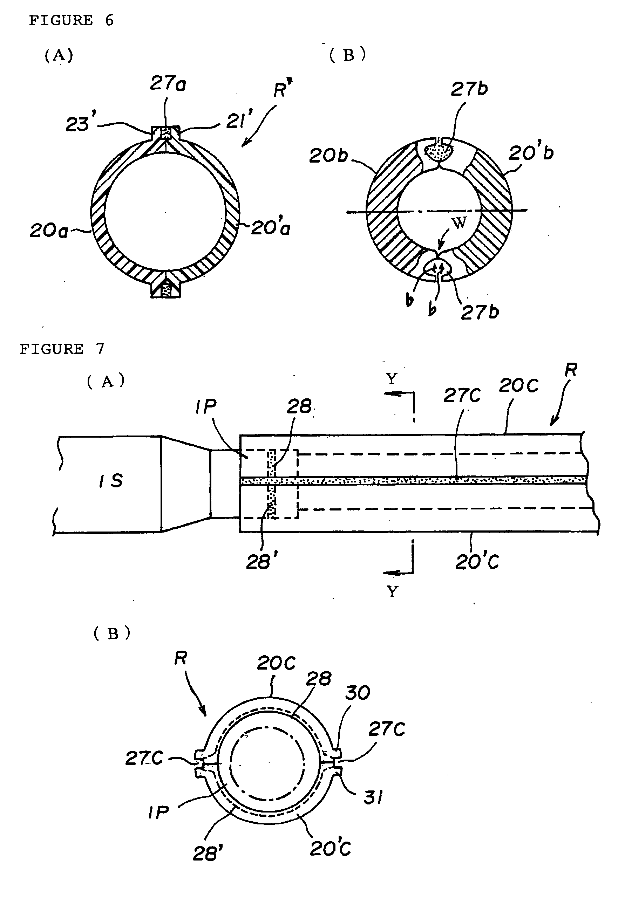

Figs. 6 (A) and (B) are different sectional views of the bodily fluid flow passage obtained by carrying out the invention;
Figs. 7 are schematic diagrams showing another bodily fluid flow passage obtained by carrying out the invention, wherein (A) is a front view showing a state where an insert article is connected to the bodily fluid flow passage, and (B) is a sectional view taken along the line Y-Y in (A);

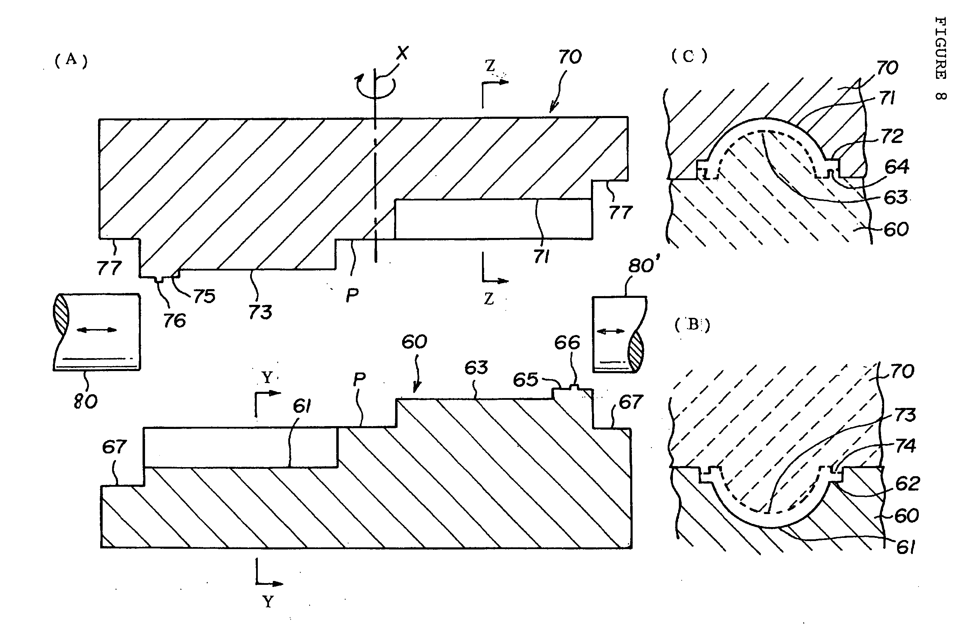

Figs. 8 are schematic diagrams showing the mold used in the invention, wherein (A) is a sectional view showing a state where the mold is opened, (B) is a sectional view taken along the line Y-Y in (A) in a state where the mold is closed, and (C) is a sectional view taken along the line Z-Z in (A) in a state where the mold is closed; and

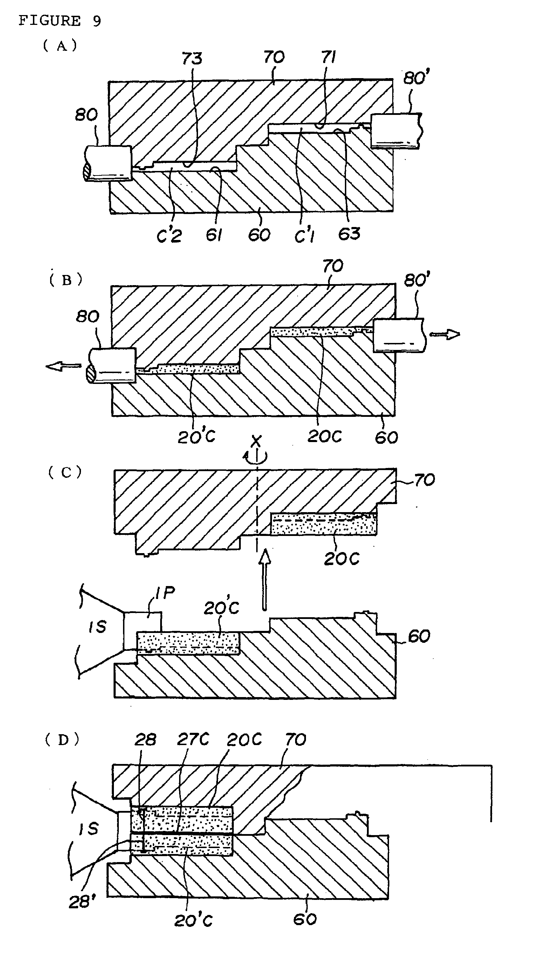

Figs. 9 are schematic diagrams of a forming example of the bodily fluid flow passage shown in Fig. 7(A) using the mold shown in Figs. 8, wherein (A) is a sectional view showing a state where the mold is clamped at the primary molding position, (B) is a sectional view showing a state where the primary molding is completed, (C) is a sectional view showing a state where the moving mold is opened, and (D) is a sectional view showing a state where the moving mold is moved to the secondary molding position and the secondary molding is completed.

### Explanation of Symbols

- T: bodily fluid purification apparatus body
- U1, U2: bodily fluid purification cassette
- R, R1, R2: bodily fluid flow passage
- PR1, PR2: pump rotor
- PT1, PT2: fluid feed pump tube
- P1, P2: roller type pump
- F1, f2: pair of box-like body
- IS: insert article
- 20, 20', 20a, 20'a, 20b, 20'b, 20c, 20'c: a pair of semi-molded articles having split-type structure
- 21, 23, 21', 23', 30, 31: flange portion
- 25: partition member
- 27, 27a, 27b, 27c: junction space

### Best Mode for Carrying Out the Invention

Embodiments of the present invention will be explained. According to the embodiment, as shown in Figs. 1 (A) and (B), the bodily fluid purification apparatus comprises a bodily fluid purification apparatus body T, and a plurality of bodily fluid purification cassettes U1, U2, ... which are selectively detachably attached to the bodily fluid purification apparatus body T. The bodily fluid purification apparatus body T has a box-like shape. The bodily fluid purification apparatus body T is provided at its front upper position with a mounting recess SB on which the bodily fluid purification cassettes U1, U2, ... are mounted. The bodily fluid purification apparatus body T is also provided at its side with a plurality of operation switches SW1, SW2, ... connected to a control apparatus. The bodily fluid purification apparatus body T is provided therein with conventionally known members such as a bubble detector which detects bubbles in a bodily fluid flow passage, a pressure detector which detects a pressure of bodily fluid in the bodily fluid flow passage, a dialysate fluid supply device, a heater, a thermometer, a conductivity meter, constituent elements of a plurality of roller type pumps, and a control apparatus.

As conventionally known, the roller type pump includes a substantially arc pump tube, and a pump rotor which is rotated and driven in the pump tube. Driving sections of the pump rotors are provided in the bodily fluid purification apparatus body T, but the pump rotors PR1 to PR4 project outward from a bottom wall of the mounting recess SB. Since the pump rotors PR1 to PR4 project, if the bodily fluid purification cassettes U1, U2, ... are mounted, as will be described in detail later, the pump rotors PR1 to PR4 are accommodated in roller type pump elements PY1 to PY4 of the bodily fluid purification cassettes U1, U2, .... Therefore, if the pump rotors PR1 to PR4 of the bodily fluid purification apparatus body B are rotated and driven, the pump tubes PT1 to PT4 provided on the side of the bodily fluid purification cassettes U1, U2, ... are sequentially crushed between a stator and the pump rotors PR1 to PR4, and bodily fluid or dialysate fluid in the pump tubes PT1 to PT4 is sent under pressure.

Other embodiments of the bodily fluid purification cassettes U1, U2, ... are shown Figs. 2 and 3. That is, an embodiment of a blood dialysis cassette U1 is shown in Fig. 2, and an embodiment of a peritoneal dialysis cassette U2 is shown in Fig. 3. As will be described later, the blood dialysis cassette U1 of the embodiment includes a plurality of dialysis elements 1 to 4 including a dialyzer 2, and four roller type pump elements, i.e. , fluid feed pump tubes PT1 to PT4, and these elements are connected through bodily fluid flow passages R1 to R7. As shown in Fig. 2 (B), the elements except the dialyzer 2 having relatively large capacities are accommodated in a container comprising upper and lower box-like bodies F1 and F2, and they are formed into a cassette or a unit.

This will be explained in more detail. In the embodiment shown in Fig. 2 (A), the blood dialysis cassette U1 includes first to fourth roller type pump elements PY1 to PY4. These roller type pump elements PY1 to PY4 respectively include pump tubes PT1 to PT4 extending about half of circumference, and stators S1 to S4 provided outside the roller type pump elements PY1 to PY4 in corresponding thereto. If the blood dialysis cassette U1 is mounted on the bodily fluid purification apparatus body T, the pump rotors PR1 to PR4 provided on the side of the apparatus body T are accommodated in the pump tubes PT1 to PT4 of the blood dialysis cassette U1. With this, the first to fourth roller type pumps P1 to P4 are constituted. Therefore, predetermined pump rotors PR1 to PR4 of the bodily fluid purification apparatus body T are rotated and driven in a predetermined direction, the pump rotors PR1 to PR4 rotate while crushing the pump tubes PT1 to PT4 against the stators S1 to S4, and blood or dialysate fluid in the pump tubes PT1 to PT4 is sent under pressure. The blood dialysis cassette U1 includes an air trap 1 for separating air in blood, a blood dialysis filter for removing toxins in blood, i.e., a dialyzer 2, a precise filter 3 for removing endotoxins which are cell body toxins, and a mesh filter 4 for removing fibrin cluster generated in blood. These dialysis elements 1 to 4 are connected through the bodily fluid flow passages R1 to R7 as will be explained in the paragraph of effect. The branch points J1 to J3 of the bodily fluid flow passages R1 to R7 may be provided with three-way valves for switching the bodily fluid flow passages R1 to R7 appropriately, but in this embodiment, the first to fourth roller type pumps P1 to P4 are appropriately actuated and stopped, thereby switching the bodily fluid flow passages R1 to R7.

Since the blood dialysis cassette U1 of the embodiment has the above-described structure, the blood dialysis cassette U1 is mounted on the bodily fluid purification apparatus body T, catheters connected to an artery and a vein of a patient are connected to connectors 6 and 7, and an operation switch SW1 for blood dialysis of the bodily fluid purification apparatus body T is pushed. Then, the selected pump rotors PR3 and PR4 are rotated, and the third and fourth roller type pumps P3 and P4 are operated. Blood is sucked from the bodily fluid flow passage R1 by the third roller type pump P3, and is discharged into the bodily fluid flow passage R2. Then, the blood passes through the air trap 1, passes through the branch point J1 from the bodily fluid flow passage R3 and is sent from the bodily fluid flow passage R4 into the dialyzer 2 under pressure, and flows downward in the dialyzer 2. Dialysate fluid is supplied from the upper supply hole 8 of the precise filter 3. The dialysate fluid flows through the dialyzer 2 from below to upward, and is discharged out from the blood dialysis cassette U1 from the upper discharge opening 9 of the dialyzer 2. The dialyzer 2 is provided with the dialysis film as in the conventional technique as described above. Therefore, while blood and dialysate fluid flow in the opposed manner, the blood is dialyzed. Then, it is sent to the mesh filter 4 under pressure through the bodily fluid flow passage R5, the branch point J3 and the bodily fluid flow passage R6. Fibrin cluster generated in the blood is removed by the mesh filter 4, pressure thereof is increased by the fourth roller type pump P4, and the blood is returned toward the vein of the patient from the bodily fluid flow passage R7 through the connector 7. When the pump rotor PR4 of the fourth roller type pump P4 opens the fluid feed pump tube PT4 during the dialysis, the fourth roller type pump P4 can be remain stopping.

According to the blood dialysis cassette U1 of the embodiment, the dialysis treatment of blood can be carried out. The blood dialysis filtering treatment, the blood filtering treatment and the priming operation for using the blood dialysis cassette can also be carried out by appropriately operating or stopping the first to fourth roller type pumps P1 to P4. These treatments are apparent for a person skilled in the art, explanation thereof will be omitted.

Fig. 3 shows the peritoneal dialysis cassette U2 as another embodiment of the bodily fluid treating cassette. The same elements as the constituent elements of the blood dialysis cassette U1 are designated with the same reference numbers and explanation thereof will be omitted. The peritoneal dialysis cassette U2 of this embodiment only has second and fourth roller type pumps P2 and P4. Therefore, the peritoneal dialysis cassette U2 is mounted on the bodily fluid purification apparatus body T, a visceral cavity catheter which is connected to visceral cavity of a patient is connected to the connector 6, and if the peritoneal dialysis operation switch SW2 of the bodily fluid purification apparatus body T is turned ON, the fourth roller type pump P4 is actuated. Dialysate fluid staying in the visceral cavity of the patient is sucked by the fourth roller type pump P4 through the bodily fluid flow passage R20, and is discharged from a weep hole 9 out from the peritoneal dialysis cassette U2 through the bodily fluid flow passage R21, the branch passage J20 and the bodily fluid flow passage R22. If other operation switch, e.g., SW3 of the bodily fluid purification apparatus body T is pushed after the dialysate fluid is discharged, then, the fourth roller type pump P4 is stopped and the second roller type pump P2 is actuated. The dialysate fluid enters the precise filter 3 from the dialysate fluid inlet opening 8, the dialysate fluid is sucked into the second roller type pump P2 from the bodily fluid flow passage R23, pressure thereof is increased, the dialysate fluid is sent to the visceral cavity of the patient from the connector 6 through the air trap 1 and the bodily fluid flow passage R24.

The present invention is to form a bodily fluid purification cassette such as the blood dialysis cassette U1 and the peritoneal dialysis cassette U2 by means of primary and secondary injection molding. That is, the invention provides a forming method for forming by means of injection molding while using, as a split-type structure, a synthetic resin hollow container in which a bodily fluid purification element is incorporated and a bodily fluid flow passage which connects the bodily fluid purification element, or provides a forming method for integrally connecting the bodily fluid purification element to the bodily fluid flow passage when the bodily fluid flow passage is to be molded.

First, an embodiment of the forming method of the bodily fluid flow passage R will be explained. Constituent elements of the bodily fluid purification cassettes U1, U2, ... are accommodated in upper and lower box-like bodies F1 and F2 as shown in Fig. 2(B) except the dialyzer 2, and it is preferable that the entire elements are as small as possible, e.g., the maximum size of the elements should be 220mm in vertical length, 300mm in lateral length and 80mm in height. A small liquid flow passage R having such a purpose is shown in Fig. 4(A), a mold or a molding example is shown in Figs. 4 (A) and 5. That is, according to the embodiment, as shown in Fig. 4 (A), first and second bodily fluid flow passages 28 and 29 comprise a pair of first and second semi-molded articles 20 and 20' of split-type structure having a semi-circular cross section, and a partition member 25 which partitions the first and second semi-molded articles 20 and 20'. The first and second semi-molded articles 20 and 20' and the partition member 25 are substantially simultaneously formed by primary forming as will be explained next, the pair of first and second semi-molded articles 20 and 20' of the split-type structure are butted against each other with the partition member 25 interposed therebetween by secondary molding, and they are integrally molded by injecting molten resin into a junction space constituted between butting or coupling flange portions.

An embodiment of the forming mold of the bodily fluid flow passage R is shown in Fig. 4(B) in a state where the mold is closed. According to the embodiment, it comprises a fixed mold 41 mounted on a fixed platen 40, and a slide mold 52 which is mounted on a movable platen 50 and which is slid in a vertical direction in Fig. 4 (B) by a piston cylinder unit 51. A recess 42 having substantially a semi-circular cross section of a predetermined size is formed above a parting line P of the fixed mold 41 for forming the second semi-molded article 20'. A shallow recess 43 is formed around the recess 42. The recess 43 is spread radially outward by a predetermined amount. This shallow recess 43 forms a coupling flange 21 on an opening or butting portion of the second semi-molded article 20'. A core 44 for forming the first semi-molded article 20 is formed below the parting line P of the fixed mold 41. The core 44 has substantially a semi-circular cross section having a predetermined size. A low core 45 is provided around the core 44. A step 26 on which a partition member 25 shown in Fig. 4 (A) is mounted is formed by this low core 45. The recess 42 and the core 44 are provided at a predetermined distance from each other in the vertical direction on the side of the parting line P of the fixed mold 41. A rectangular recess 46 having a substantially rectangular cross section is formed on the side of the fixed mold 41 therebetween. The rectangular recess 46 forms a partition member 25. Conventionally known sprues 47 and runner 48 are provided on the side of the fixed platen 40 and the fixed mold 41. The sprues 47', 47' and 47' are opened into a recess 42, a rectangular recess 46 and a recess of a later-described slide mold 52 through gates.

Fig. 4 (B) is a sectional view showing a state where the slide mold 52 is closed with respect to the fixed mold 41 at a primary molding position. As shown in this sectional view, a core 53 corresponding to the recess 42 of the fixed mold 41 is formed on the side of the parting line P of the slide mold 52. The core 53 is smaller than the recess 42 of the fixed mold 41 by a predetermined amount. With this, the second semi-molded article 20' having the predetermined thickness is formed. A small core 54 is provided around the core 53. A step 22 on which the partition member 25 shown in Fig. 4(A) is mounted is formed by the small core 54. A recess 55 having substantially a semi-circular cross section of a predetermined size is formed below the parting line P of the slide mold 52. The recess 55 forms the first semi-molded article 20. A shallow recess 56 spreading radially outward by a predetermined amount is formed around the recess 55. A coupling flange portion 23 is formed on the first semi-molded article 20 by the shallow recess 56.

Next, a forming example for forming the bodily fluid flow passage R4 shown in Fig. 4(A) using the molds 41 and 52 will be explained. The slide mold 52 is moved to the primary molding position shown in Fig. 4 (B) and the slide mold 52 is clamped. With this, a second cavity C2 for forming the second semi-molded article 20' is constituted by the recess 42 of the fixed mold 41 and the core 53 of the slide mold 52. At that time, a cavity for forming the coupling flange 21 is also formed in an opening or a butting portion of the second semi-molded article 20'. A first cavity C1 for forming the first semi-molded article 20 is constituted by the core 44 of the fixed mold 41 and the recess 55 of the slide mold 52. At that time, a cavity for forming the coupling portion, i.e., the flange portion 23 is also constituted in the opening of the first semi-molded article. A cavity C3 for forming the partition member 25 is constituted by a rectangular recess 46 of the fixed mold 41 and a surface of the parting line P of the slide mold 52. Fig. 4(B) shows a state where such cavities C1 to C3 are constituted.

Molten resin is injected from the injection unit into the cavities C1 to C3 through the sprue 47, the runner 48, the sprues 47', 47' and 47' and gates (not shown in Figs. 4 or 5). The pair of first and second semi-molded articles 20 and 20' and the partition member 25 are molded by the primary molding. Fig. 5 (A) shows a state where they are primary molded. The slide mold 52 is opened as shown in Fig. 5(B) after they are cooled and solidified for a while. Due to differences of shape and size of the molded articles, the first semi-molded article 20 remains on the side of the recess 55 of the slide mold 52 and the second semi-molded article 20' and the partition member 25 remain on the side of the recesses 42 and 46 of the fixed mold 41 and the mold is opened.

The partition member 25 is taken out by a robot for example and is inserted into the second semi-molded article 20'. That is, an end of the partition member 25 is placed on the coupling step 22 of the second semi-molded article 20'. The slide mold 52 is driven to the secondary molding position shown in Fig. 5 (C) by the piston cylinder unit 51, and the molds 41 and 52 are clamped. Fig. 5(D) shows the clamped state. Next, molten resin is injected from a secondary molding runner 27'' into a junction space 27 between the flange portions 21 and 23 as shown in Fig. 5(E) in enlarged scale. With this, the first and second semi-molded articles 20 and 20' and the partition member 25 are integrally formed. If the slide mold 52 is opened after it is cooled and solidified, the bodily fluid flow passage R formed of the first and second flow passages 28 and 29 as shown in Fig. 4(A) is molded. Other bodily fluid flow passages R1 to R23 are also molded in the same manner.

In this embodiment, since the partition member 25 is provided, two first and second flow passages 28 and 29 are formed by the partition member 25, but it is of course possible to eliminate the partition member 25 so that the bodily fluid flow passage comprising one flow passage can be formed by the first and second semi-molded articles 20a and 20'a. Fig. 6 (A) is a sectional view of the bodily fluid flow passage R' comprising one flow passage. According to the embodiment, although the structure of the flange portion or the junction space 27a is different, it is apparent that coupling flange portions 21' and 23' of such structure can also be formed by changing the shapes of molds. Fig. 6(B) shows another embodiment in which the shape of the coupling portion is different. In Fig. 6(B), a lower portion shows a shape of a coupling space 27b before the secondary molding, and an upper portion shows a state where the secondary molding is completed. According to the embodiment, if openings or coupling portions of the pair of first and second semi-molded articled 20b and 20'b are butted against each other, the cross section of the junction space 27b has substantially a rounded triangular shape. An inner side of the butting portion is recessed radially outward such that its cross section has substantially a triangular shape. The recessed portion is shown with the arrow W. Therefore, if secondary molding molten resin is injected into the junction space 27b, the butting portion receives forces in the directions of arrows b and b and the butting portion is deformed. By such deformation, the gap of the butting portion becomes 2µm or less although it depends on secondary molding resin pressure, material and shape of the butting portion and the like. In Fig. 6(B), the upper portion shows a state where the inner portions of the butting portion are in tight contact with each other.

Next, a molding example in which separately obtained insert articles, i.e., bodily fluid purification elements such as the air trap 1, the precise filter 3 and the fluid feed pump tubes PT1 to PT4 which are constituent elements of the roller type pump when the bodily fluid flow passage R is molded are integrally connected to the bodily fluid flow passage R will be explained. Figs. 7 (A) and (B) show an example of a molded article in which an insert article is integrally formed on the bodily fluid flow passage R. That is, according to this molded article, a pair of first and second semi-molded articles 20c and 20'c of split-type structure are molded such that their openings or butting portions have flange portions 30 and 31 by primary molding, a bodily fluid access opening IP of an insert article IS is placed on the second semi-molded article 20'c, the first semi-molded article 20c is superposed thereon and clamped, molten resin is injected into the junction space 27c constituted between the flange portions 30 and 31 by the secondary molding, and they are integrally formed together. When they are integrally formed together, the secondary molding molten resin is also charged between an outer periphery of the bodily fluid access opening IP of the insert article IS and an inner periphery of the first and second semi-molded articles 20c and 20' c as will be described in detail later. With this, the insert article IS and the bodily fluid flow passage R are liquid-tightly connected to each other.

Figs. 8 show an embodiment of the mold used for carrying out the forming method of the insert article. The mold of this embodiment includes a fixed mold 60 and a moving mold 70 which is opened and closed with respect to the fixed mold 60 and which is rotated around an axis X. Fig. 8(A) is a sectional view showing a state where the moving mold 70 is opened, Fig. 8(B) is a sectional view taken along the line Y-Y in Fig. 8(A) in a state where the moving mold 70 is clamped, and Fig. 8(C) is a sectional view taken along the line Z-Z in Fig. 8(A) in a state where the moving mold 70 is clamped. As shown in Fig. 8(B), a recess 61 having a substantially semi-circular cross section forming the second semi-molded article 20'c is formed on the side of the parting line P of the fixed mold 60. A core 63 having substantially a semi-circular cross section is provided on the right side of the recess 61. The core 63 is for forming the first semi-molded article 20c. As shown in Fig. 8(B), a shallow small recess 62 is formed around the recess 61. A coupling flange 31 is formed on the butting portion of the first semi-molded article 20c by the small recess 62. As shown in the sectional view of Fig. 8(C), a low small core 64 is formed around the core 63 of the fixed mold 60 at a predetermined distance from the core 63. A step forming core 65 having a predetermined width is provided close to a right end of the core 63. The height of the step forming core 65 is the same as the thickness of the bodily fluid access opening IP of the insert article IS. With this, a step having an increased diameter toward the inner peripheral surface of the first semi-molded article 20c is formed, and the bodily fluid access opening IP of the insert article IS is fitted. A small core 66 is provided on a tip of the step forming core 65. With this, a semi-circumferential groove 28' into which molten resin is charged at the time of secondary molding is formed.

The moving mold 70 pair off with the fixed mold 60. A core 73 having substantially a semi-circular cross section is provided on the side of the parting line P. The core 73 is for forming the second semi-molded article 20'c. A recess 71 having substantially a circular cross section is provided on the right side of the core 73. The recess 71 forms the first semi-molded article 20c. As shown in Fig. 8(C), a small shallow recess 72 is formed around the recess 71. With this small shallow recess 72, a coupling flange 30 is formed on the butting portion of the first semi-molded article 20c. As shown in the sectional view of Fig. 8(B), the core 73 of the moving mold 70 is also provided with a low small core 74 at a predetermined distance from the core 73. A step forming core 75 having a predetermined width is formed close to a left end of the core 73. The height of the step forming core 75 is the same as the thickness of the bodily fluid access opening IP of the insert article IS. With this, a step whose diameter is increased is formed in the inner peripheral surface of the second semi-molded article 20'c, and the bodily fluid access opening IP of the insert article IS is fitted on it. The step forming core 75 is provided at its top with a small core 76. With this, a semi-circumferential groove 28 into which molten resin is charged at the time of secondary molding is formed. Both ends of the fixed mold 60 and the moving mold 70 are formed with relief notches 67, 67, 77 and 77 used when the insert article IS is mounted. This embodiment also has a pair of moving cores 80 and 80' used at the time of primary molding explained in the paragraph of effect.

A molding example of a molded article as shown in Figs. 7 will be explained using the molds 60 and 70 and moving cores 80 and 80. Molds are clamped at the primary molding position shown in Fig. 8(A). The pair of moving cores 80 and 80' are driven to positions shown in Fig. 8(A). With this, a cavity C'2 for molding the second semi-molded article 20'c is constituted by the recess 61 of the fixed mold 60, the core 73 of the moving mold 70 and the moving core 80. A cavity C'1 for molding the first semi-molded article 20c is constituted by the core 63 of the fixed mold 60, the recess 71 of the moving mold 70 and the moving core 80'. Although it is not illustrated in Fig. 9(A), a cavity for integrally molding flanges 30 and 31 on the first and second semi-molded articles 20c and 20'c, and a cavity for molding a step on which the bodily fluid access opening IP of the insert article IS is mounted are also constituted by clamping. A cavity for molding resin passages 28 and 28' into which molten resin is charged at the time of secondary molding is also constituted in an inner peripheral surfaces of steps of the first and second semi-molded articles 20c and 20'c.

Although it is not illustrated in the drawing, molten resin for primary molding is injected from the fixed mold 60 toward the cavities C'1 and C'2 from the injection unit. By this primary molding, as shown in Fig. 9(B), the pair of first and second semi-molded articles 20c and 20'c of the split-type structure are molded. The moving cores 80 and 80' are retreated and the moving mold 70 is opened after they are cooled and solidified. The second semi-molded article 20'c is remained in the fixed mold 60 and the first semi-molded article 20c is remained in the moving mold 70 and they are opened. Fig. 9(C) shows this opened state. The separately obtained insert article IS is grasped by revolute robots or the like and the bodily fluid access opening IP is inserted into the step of the second semi-molded article 20' c. The moving mold 70 is rotated toward the secondary molding position around the axis X through 180°. If it is rotated and moved to the secondary molding position, the first semi-molded article 20c is aligned with the second semi-molded article 20'c. That is, openings of the first and second semi-molded articles 20c and 20'c are butted against each other. At this position, the molds are clamped as shown in Fig. 9(D). As shown in Fig. 7(B) in detail, a secondary molding charging space 27c is constituted in the butting portions of the first and second semi-molded articles 20c and 20'c by the flange portions 30 and 31. Resin passages 28 and 28' which are in communication with the charging space 27c are also formed. Secondary molding molten resin is injected. The molten resin is charged into the charging space 27c and the resin passages 28 and 28', and the pair of first and second semi-molded articles 20c and 20'c and the insert article IS are liquid-tightly integrally formed together. After they are cooled and solidified, the moving mold is opened, and a molded article in which the bodily fluid flow passage R is integrally formed with the insert article IS is taken out. The molding operation is carried out in the same manner.

The present invention can be carried out in various forms. For example, although the moving mold 70 rotates in the above embodiment, the moving mold 70 can also slide as explained with reference to Fig. 4(B). When the first and second semi-molded articles 20c and 20'c of the split-type structure are to be molded, it is apparent that the shape of the butting portion can be carried out as shown in Fig. 6(B).

In the above explanation, the mold used for molding the bodily fluid flow passage of the bodily fluid purification cassette such as the blood dialysis cassette and the peritoneal dialysis cassette, and a mold which integrally forms the insert article when the bodily fluid flow passage is to be molded are different from each other, but it is apparent that they can be molded using one mold. It is also apparent that a relatively large recess is separately formed in the parting line of such a mold, an insert article is put into the recess at the time of primary molding, and it can be preheated.

As shown in Fig. 2(B), the bodily fluid purification cassette formed in this manner is accommodated in the pair of box-like bodies F1 and F2 and formed into a cassette. That is, using the fixed mold and the slide mold, the bodily fluid flow passage described in the primary molding process is formed as a split body of split-type structure. After the primary molding, one of the split halves is remained in the slide mold, the other split half is remained in the fixed mold and the slide mold is opened, and the slide mold is slid to the secondary molding position. Then, the bodily fluid purification element is inserted, the split halves of the split-type structure are butted against each other and they are clamped. At that time, a cavity for molding the pair of box-like bodies F1 and F2 is also formed simultaneously in the fixed mold and the slide mold. Next, if secondary molding molten resin is injected, the butting portions of the split halves of the split-type structure are connected to each other. As shown in Fig. 7 (A), the bodily fluid flow passage R and the bodily fluid purification element are integrally formed together. At the same time, the box-like bodies F1 and F2 are also formed. The slide mold is opened such that the box-like bodies F1 and F2 are remained in the molds, respectively. The integrally formed bodily fluid flow passage R and the bodily fluid purification element are inserted into the box-like body F1, and the slide mold is slid to a position where the openings of the box-like bodies F1 and F2 are opposed to each other. Lastly, the molds are clamped, and molten resin is injected into butting portions of the box-like bodies F1 and F2. As a result, the box-like bodies F1 and F2 are coupled to each other through the butting portions TS and they are formed into a cassette as shown in Fig. 2(B). They can be cassetted in this manner, but the dialyzer 2 which is relatively large in size can also be added onto the box-like body F1 at a later stage. If the dialyzer 2 is added later in this manner, necessary portions such as the bodily fluid flow passage are also added later.

### Industrial Applicability

It is possible to inexpensively produce, in a sanitary manner, a bodily fluid purification cassette which is reduced in size as small as possible, which is mounted on and cooperates with a bodily fluid treating apparatus body to carry out bodily fluid purification, in which a bodily fluid purification element suitable for use such as blood dialysis or peritoneal dialysis, and a bodily fluid flow passage which is liquid-tightly connected to the bodily fluid purification element are provided.

## Claims

1. A forming method of a bodily fluid purification cassette in which the bodily fluid purification cassette is selectively attached to a bodily fluid purification apparatus body comprising a bubble detector which detects bubbles in a flow passage, a pressure detector which detects a pressure of bodily fluid in the flow passage, a dialysate fluid supply device, a heater, a thermometer, a conductivity meter, a pump rotor of at least one roller type pump, and a control apparatus,
the bodily fluid purification cassette incorporates:
basic elements comprising at least a bodily fluid flow passage, an air trap and a fluid feed pump tube;
a selective element selected from bodily fluid purification elements comprising a dialyzer, a blood filter, a precise filter, a mesh filter, a plasma exchange module and an immunoadsorbent cylinder; and
a bodily fluid flow passage which connects the basic element and the selective element with each other, and
if the bodily fluid purification cassette which incorporates these elements is attached to the bodily fluid purification apparatus body, the pump rotor of the bodily fluid purification apparatus body cooperates with the fluid feed pump tube of the basic element to constitute a roller type pump, so that desired bodily fluid purification such as blood dialysis, peritoneal dialysis, plasma exchange and immune adsorption can be carried out, wherein
a synthetic resin hollow container in which the basic element, the selective element and the bodily fluid flow passage are incorporated, and the bodily fluid flow passage are formed such that a cross section of a split-type structure has a predetermined shape by primary molding, the split-type structure is integrally formed by secondary molding to form the bodily fluid purification cassette in which the basic element, the selective element and the bodily fluid flow passage are incorporated in the synthetic resin hollow container,
and when the bodily fluid purification cassette is formed,
concerning the bodily fluid flow passage, a pair of first and second semi-molded articles having substantially semi-circular cross section of the split-type structure and a partition member having substantially a plate-like shape are injection molded by primary molding such that the pair of first and second semi-molded articles are provided at their butting portions with coupling flanges,
the butting portions of the pair of first and second semi-molded articles sandwich the partition member by secondary molding, molten resin is injected into a junction space constituted by the flange portions of the pair of first and second semi-molded articles, the pair of first and second semi-molded articles and the partition member are liquid-tightly integrally formed together, and the bodily fluid flow passage comprising two flow passages is formed.

2. A forming method of a bodily fluid purification cassette in which the bodily fluid purification cassette is selectively attached to a bodily fluid purification apparatus body comprising a bubble detector which detects bubbles in a flow passage, a pressure detector which detects a pressure of bodily fluid in the flow passage, a dialysate fluid supply device, a heater, a thermometer, a conductivity meter, a pump rotor of at least one roller type pump, and a control apparatus,
the bodily fluid purification cassette incorporates:
basic elements comprising at least a bodily fluid flow passage, an air trap and a fluid feed pump tube;
a selective element selected from bodily fluid purification elements comprising a dialyzer, a blood filter, a precise filter, a mesh filter, a plasma exchange module and an immunoadsorbent cylinder; and
a bodily fluid flow passage which connects the basic element and the selective element with each other, and
if the bodily fluid purification cassette which incorporates these elements is attached to the bodily fluid purification apparatus body, the pump rotor of the bodily fluid purification apparatus body cooperates with the fluid feed pump tube of the basic element to constitute a roller type pump, so that desired bodily fluid purification such as blood dialysis, peritoneal dialysis, plasma exchange and immune adsorption can be carried out, wherein
a synthetic resin hollow container in which the basic element, the selective element and the bodily fluid flow passage are incorporated, and the bodily fluid flow passage are formed such that a cross section of a split-type structure has a predetermined shape by primary molding, the split-type structure is integrally formed by secondary molding to form the bodily fluid purification cassette in which the basic element, the selective element and the bodily fluid flow passage are incorporated in the synthetic resin hollow container,
and when the bodily fluid purification cassette is formed,
the bodily fluid flow passage is formed by primary molding such that the bodily fluid flow passage includes a pair of first and second semi-molded articles of split-type structure having substantially a semi-circular cross section, butting portions thereof have flange portions and ends of the flange portions have coupling step having increased diameter,
connected portions of the basic element and the selective element are inserted into the coupling steps of the first and second semi-molded articles and the molds are clamped,
molten resin is injected by secondary molding into a junction space constituted by the flange portions of the first and second semi-molded articles, and into a space between outer peripheries of the connected portions and inner peripheries of the coupling steps of the first and second semi-molded articles, and
the basic element and the selective element are liquid-tightly connected to the bodily fluid flow passage.

3. The forming method of the bodily fluid purification cassette according to claim 1 or 2, wherein the primary molding and the secondary molding are carried out using the same mold, and the bodily fluid purification cassette is formed.

4. The forming method of the bodily fluid purification cassette according to claim 2, wherein the basic element and the selective element are preheated in the same mold before the secondary molding.

5. The forming method of the bodily fluid purification cassette according to any one of claims 1 to 4, wherein when the first and second semi-molded articles are to be formed by the primary molding, they are formed so that a cross section shape of the junction space constituted by the flange portions by butting the butting portions against each other has substantially a triangular shape whose angle portions are rounded, and so that an inner peripheral surface side of the butting portion is recessed radially outward such that its cross section has substantially a triangular shape.

6. The forming method of the bodily fluid purification cassette according to any one of claims 1 to 5, wherein the basic element, the selective element and the bodily fluid flow passage are formed such that they can be accommodated in a synthetic resin hollow container having a size equal to or less than 220mm in vertical length, 300mm in lateral length and 80mm in height.
